# EUROPEAN PATENT APPLICATION

(11) **EP 1 568 997 A2**
(43) Date of publication of application: **31.08.2005**
(21) Application number: 05002152.6
(22) Date of filing: 02.02.2005
(51) Int. Cl.: G01N 33/78

(54) **Surface for detection of interaction between substances and corresponding sensor chip, sensing device and detection method**

(30) Priority: 09.02.2004 JP 2004031824; 17.09.2004 JP 2004270828
(71) Applicant: SONY CORPORATION, Tokyo (JP)
(72) Inventor: Watanabe, Yuuki c/o Sony Corproation, Tokyo (JP)
(74) Representative: Körber, Martin Hans

(57) **Abstract**

Disclosed herein is a surface (1) capable of detecting interaction between substances. This surface has double-stranded DNA (2) which forms the complimentary linkage with single-stranded DNA having one end thereof fixed to the surface, such that the double-stranded DNA (2) dissociates into single-stranded DNA (21, 22) in response to the interaction. Disclosed also herein are a sensor chip and a sensing device, which have the detecting surface as a constituent, and a method for detecting interaction between substances.

## Description

### BACKGROUND OF THE INVENTION

The present invention relates a technique of detecting interaction between substances. More particularly, the present invention relates to a detecting surface designed to detect interaction between substances, a sensor chip and a sensing device equipped with the detecting surface as a constituent, and a detecting method.

The recent remarkable development of molecular biology, nanotechnology for microfabrication, and bioinformatics relies on the newly developed technique of detecting interaction between substances on a very small surface based on various measuring principles. (This technique will be referred to as "sensor technique" hereinafter.)

The sensor technique is widely used for analysis of bioinformation, such as genomics, transcriptome analysis for genome to transcription, proteome analysis for expression proteins which are translated and produced in living organisms and cells, metabolome analysis for metabolism, and signalome analysis for signals in living organisms. It is establishing itself as a powerful tool in such fields as innovative drug development, clinical diagnosis, pharmacological genomics, and forensic medicine.

There are known several sensor techniques capable of detecting interaction between substances as exemplified in the following. The first technique relates to the integrated substrate for bioassay (called DNA chip or DNA microarray), which has a predetermined number of DNA molecules arranged on a substrate by the microarray technology. (See Patent Documents 1 and 2.) This technique permits analysis of hybridization with the help of a variety of and a large number of oligo DNA strands or cDNA (complementary DNA) strands which are integrated on a glass or silicon substrate. Consequently, it is used for analysis of gene mutation, SNPs (Single Nucleotide Polymorphism) analysis, and analysis of gene expression frequencies.

The second technique relates to the protein chip (or protein array chip), which is composed of a substrate of microchip and purified protein arrayed on the surface of the substrate. The protein chip is capable of analyzing interaction between two proteins or between a protein and other chemical substance, although it might deteriorate the activity of the immobilized protein. (See Patent Documents 3 and 4.)

The third technique is based on the principle of surface plasmon resonance (SPR), which permits real-time analysis of interaction between various substances. (See Patent Documents 5 and 6.) This SPR sensor is composed of three layers functioning as absorber, substrate, and prism. It is so designed as to determine interaction between substances by means of the change in critical angle of total reflection of incident ray. What actually contributes to the change in critical angle of total reflection is the change in dielectric constant induced by interaction. It has recently been reported that this SPR sensor permits screening of endocrine disrupting chemicals in the following manner. The sensor chip has double-stranded DNA fixed thereto. The double-stranded DNA has a hormone response element in the promoter region of the estrogen responsive gene. The sensor chip is given estrogen receptor α and interaction between the hormone response element, and the estrogen receptor α is determined in real time without labeling. (See non-patent document 1.)

The fourth technique is for analyzing the change in structure of biopolymer by measuring the change in frequencies of a quarts oscillator. (See Patent Document 7.) Technique for analyzing interaction between receptor and ligand by the measurement is proposed. (See Paten Document 8.)

### [Patent Document 1]

Japanese Translations of PCT for Patent No. Hei 4-505763

### [Patent Document 2]

Japanese Translations of PCT for Patent No. Hei 10-503841

### [Patent Document 3]

Japanese Patent laid-open No. 2003-130877

### [Patent Document 4]

Japanese Patent laid-open No. 2003-155300

### [Patent Document 5]

Japanese Translations of PCT for Patent No. Hei 4-501462

### [Patent Document 6]

Japanese Translations of PCT for Patent No. Hei 11-512518

### [Patent Document 7]

Japanese Patent laid-open No. 2003-083864

### [Patent Document 8]

Japanese Patent laid-open No. 2003-083973

### [Non-patent Document 1]

BUNSEKI KAGAKU, vol. 51, No. 6, pp. 389-396 (2002)

### SUMMARY OF THE INVENTION

The above-mentioned sensor technique is designed to detect the interaction between an immobilized substance for detection and a target substance that takes place on a surface prepared for detection. It permits accurate detection if a good choice is made for the principle of measurement, which suits best to the characteristic properties of substance and the object of analysis.

However, there is a case where sensitive detection of interaction is difficult owing to the structure and molecular size of the substance to be detected. Coping with this situation is one of the technical problems to be solved. This is true for a low-molecular weight substance (such as hydrophobic hormone), which changes very little in mass and dielectric constant due to interaction, making accurate detection difficult. Under these circumstances, there is a strong demand for a technique of detecting accurately and sequentially a hormone (or hormone-like substance) as a substance to perform signal transmission, in the fields of genomics, proteomics, medicine, innovative drug development, and detection of environmental hormone.

It is a major object of the present invention to provide a sensor technique capable of accurate measurement for interaction between low-molecular weight substances. This object is achieved by creating a new assay system on a detecting surface.

The present invention is directed to a detecting surface for detection of interaction between substances. The detecting surface includes double-stranded DNA forming the complimentary linkage with single-stranded DNA having one end thereof fixed to the detecting surface, such that the double-stranded DNA dissociates into single-stranded DNA in response to the interaction. The present invention is directed also to a sensor chip for interaction, which has at least the detecting surface defined above. The present invention is directed also to a sensing device having the detecting surface defined above and a means of detecting the dissociation of the double-stranded DNA into the single-stranded DNA, which occurs on the detecting surface.

The term "detecting surface" as used in the present invention means the surface of a substrate, granules, film, or fiber, which functions as the region or space in which interaction between substances takes place, the region, or space on the surface.

The detecting surface according to the present invention is technically characterized in effectively utilizing the phenomenon that double-stranded DNA dissociates into single-stranded DNA. In other words, this detecting surface constitutes an assay system entirely different from the conventional DNA chip in which single-stranded DNA is formed into double-stranded DNA by hybridization to investigate gene expression and the like.

Hybridization on a DNA chip is poor in efficiency on account of the steric hindrance, which results from DNA strands forming a randomly coiled high-order structure. Moreover, it involves an unavoidable problem with mishybridization, which decreases the accuracy of detection. By contrast, dissociation of double-stranded DNA into single-stranded DNA is free of such problems, and the assay based on this principle has the advantage of easy operation and easy adjustment of assay conditions.

The term "interaction" as used in the present invention broadly embraces noncovalent bond, covalent bond, hydrogen bond, chemical bond, and dissociation. It also includes interaction that takes place between "a target substance present on the detecting surface" and "a detecting substance binding to a specific site of sequence of previously immobilized double-stranded DNA" when a sample is added, injected, or infused into the region on the detecting surface.

The term "target substance" broadly embraces substances that will be involved in interaction or substance to be screened that might be involved in interaction. It includes, for example, hormones and endocrine disrupting chemicals.

The term "hormone" is defined as a substance produced and secreted by specific cells in response to internal and external information of living organisms and which transports such information to other cells through body fluid. Hormones are classified as follows according to the receptor on which they act.
- Those which act on the receptor of cell membrane penetrating type (such as protein-peptide hormone and catecholamine).
- Those which act on the receptor in cells (such as steroid hormone).
- Those which act directly on the receptor in the cell nucleus (such as thyroid hormone, retinoids, and vitamin D) .

The term "endocrine disrupting chemical" denotes any substance called "environmental hormone" which acts on living organisms in the same way as estrogen (human sex hormone) or the like. The chemical substance enters living organisms to bind to the hormone receptor to which hormones originally present in living organisms should bind, thereby producing a hormone-like effect (agonist action) or inhibiting the original hormone effect (antagonist action). For example, bisphenol, nonyphenol, and DDT are known to bind to he estrogen receptor, thereby reacting like estrogen. Additional examples include dioxins (which produce an antiestrogen action), vinclozolin and DDE (which produce an antiandrogen action), and TCDD and PCB (which disrupt thyroid hormone). Refer to "Application of DNA Chip, II" (pp. 93-95), compile by K. Matsunaga, published by C.M.C.

The "detecting substance" denotes any substance that produces a direct or indirect interaction with the above-mentioned target substance. The detecting substance includes at least ligand-independent hormone receptors and ligand-dependent hormone receptors. In other words, the ligand-independent hormone previously binds directly or indirectly to a specific site of sequence of double-stranded DNA at the start of assay regardless of interaction with the target substance (ligand). The ligand-dependent hormone includes those substances, upon interaction with the target substance (ligand), which change in three-dimensional structure and bind directly or indirectly to a specific site of sequence. The "detecting substance" includes the transcription activation factor. Dissociation from double-stranded DNA into single-stranded DNA may manifest itself as the change of state resulting from transcription activation.

A "specific site of sequence" in double-stranded DNA implies a site of sequence to which the detecting substance can bind. It includes the hormone response element in the promoter region upstream from the hormone responsive gene. The hormone receptor functioning as the detecting substance binds to this hormone response element.

"Double-stranded DNA" fixed to the detecting surface is "a complementary conjugate of deoxyribonucleic acid" which has been artificially modified or adjusted such that double-stranded DNA dissociates into single-stranded DNA when the detecting substance (such as hormone receptor) binds to the specific site of sequence in the double-stranded DNA. It may also be "a complementary conjugate of deoxyribonucleic acid" which has been artificially modified or adjusted such that dissociation into single strands takes place in response to the transcription activation signal brought by the detecting substance.

The present invention may cover an embodiment in which the double-stranded DNA has an intercalator (such as fluorescent intercalator) inserted thereinto. When the double-stranded DNA dissociates into single-stranded DNA, the fluorescent intercalator releases itself on the detecting surface, thereby changing in its color development. Incidentally, the "fluorescent intercalator" is a fluorescent substance inserted into the complimentary conjugate of the double-stranded DNA owing to its binding characteristics.

The present invention may also cover an embodiment in which the end of the DNA strand fixed to the detecting surface is labeled with a fluorescent substance (such as a fluorescent dye). When the double-stranded DNA dissociates into single-stranded DNA, the fluorescent substance releases the labeled DNA strand on the detecting surface, thereby changing in its fluorescent intensity.

The present invention may also cover an embodiment in which the end of the DNA strand fixed to the detecting surface is labeled with a dielectric substance. When the double-stranded DNA dissociates into single-stranded DNA, the distance between the dielectric substance and the detecting surface changes, leading to change in dielectric constant. "Dielectric substance" undergoes electric polarization in an electrostatic field but does not produce continuous current.

The present invention may also cover an embodiment in which a complex composed of the target substance and the intercalator binds to the complimentary base pair of the double-stranded DNA. In this case, a typical example of the target substance is hormone. The intercalator may be either fluorescent one or non-fluorescent one.

The present invention may also be directed to a method of detecting an interaction between substances. The method includes a step of fixing double-stranded DNA to a detecting surface, a step of causing the double-stranded DNA to dissociate into the single-stranded DNA in response to interaction between substances, and a step of detecting the dissociation.

The method according to the present invention may be so modified as to additionally include a step of binding a conjugate composed of a target substance (hormone, for example) to be detected and an intercalator to the site of the complementary base pair of the double-stranded DNA, and a step in which the conjugate releases itself into the medium on the detecting surface when the double-stranded DNA dissociates into the single-stranded DNA, and a step in which the target substance constituting the released conjugate promotes the dissociation through combination with a receptor.

According to the modified embodiments, a large number of molecules of double-stranded DNA are made to sequentially dissociate into single strands by the process including regularly fixing the molecules of double-stranded DNA onto the detecting surface, intercalating the conjugate into the complementary base pair of double-stranded DNA using the intercalator characteristics, and introducing the target substance to be detected into the double-stranded DNA. The sequential dissociation into single strands exponentially amplifies the initial signal to be measured, thereby allowing sensing with high sensitivity.

Incidentally, the principle of measurement for detection is not specifically restricted. In some cases, it is desirable to employ the principle of surface plasmon resonance (abbreviated as SPR hereinafter). It is also possible to use any other optical principles or a principle for detecting the change in frequencies of a quarts oscillator.

To be concrete, detection may be accomplished by the procedure designed to detect, based on the principle of surface plasmon resonance, the change in dielectric constant that occurs when the distance between the detecting surface and the dielectric substance labeling the end of the fixed DNA strand of the double-stranded DNA changes as the double-stranded DNA dissociates into the single-stranded DNA.

The detection of interaction by the method of the present invention may be accomplished by optically measuring either of the following two items (1) or (2).
(1) Change in intensity of fluorescence of the fluorescent substance labeling the double-stranded DNA.
(2) Change in color development of the fluorescent intercalator inserted into the double-stranded DNA.

Thus, the present invention permits to measure with high accuracy even if any level of interaction is involved with low-molecular weight substances.

### BRIEF DESCRIPTION OF THE DRAWINGS

These and other objects of the invention will be seen by reference to the description, taken in connection with the accompanying drawing, in which:
Fig. 1 is a schematic diagram illustrating the concept of one fundamental mechanism governing the present invention;
Fig. 2 is a schematic diagram illustrating the concept of another fundamental mechanism governing the present invention;
Fig. 3 is a schematic diagram illustrating the concept of the first embodiment suitable for detection of dissociation of the double-stranded DNA into the single-stranded DNA;
Fig. 4 is a schematic diagram illustrating the concept of the second embodiment for the same purpose as above;
Fig. 5 is a schematic diagram illustrating the concept of the third embodiment for the same purpose as above;
Fig. 6A is a schematic diagram illustrating how a hormone (such as glucocorticoid) works;
Fig. 6B is a schematic diagram illustrating how a hormone (such as thyroid hormone) works;
Fig. 7 is a schematic diagram showing how a hormone-receptor complex acts on GRE in DNA, thereby causing the DNA to dissociate into single strands;
Fig. 8 is a schematic diagram showing the molecules of double-stranded DNA fixed to the detecting surface at regular intervals;
Fig. 9 is a schematic diagram showing how the hormone-receptor complex acts on the hormone response element (GRE), thereby causing the double-stranded DNA to dissociate into single strands and release the conjugate;
Fig. 10 is a schematic diagram showing what occurs after the conjugate released from a first double-stranded DNA has intercalated itself into the complementary base pair in a second double-stranded DNA;
Fig. 11 is a schematic diagram showing how the target substance (such as corticoid) constituting the conjugate released from the double-stranded DNA forms the hormone-receptor complex, which subsequently acts on GRE of another double-stranded DNA;
Fig. 12 is a simplified diagram showing the basic structure of the sensing device pertaining to the present invention.

### DETAILED DESCRIPTION OF THE PREFERRED EMBODIMENTS

The preferred embodiments of the present invention will be described below with reference to the accompanying drawings.

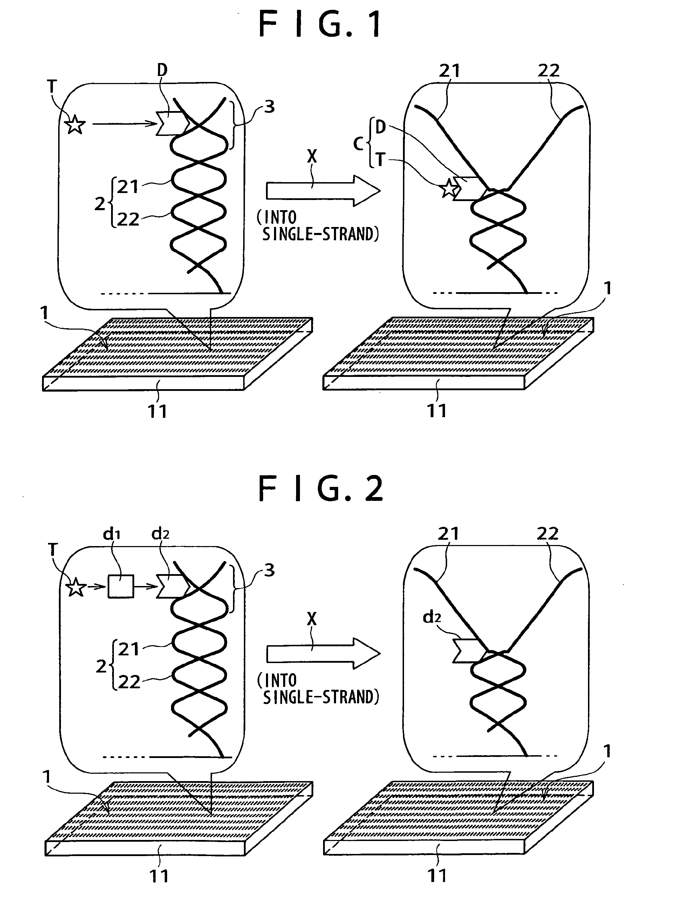

Fig. 1 is a schematic diagram illustrating the concept of one fundamental mechanism governing the present invention. Fig. 2 is a schematic diagram illustrating the concept of another fundamental mechanism governing the present invention. Incidentally, the arrow "X" in Figs. 1 and 2 indicates transition from a double-stranded DNA to a single-stranded DNA. (This applies to other figures.)

The reference numeral 1 in Figs. 1 and 2 denotes one embodiment of the detecting surface pertaining to the present inventions. The illustrated detecting surface 1 to detect interaction is a surface (and some regions or space thereon) of a substrate 11 formed from glass, transparent synthetic resin, amorphous carbon, or the like. Incidentally, the detecting surface 1 may be a thin metal film (of gold, for example) formed by vapor deposition in the case where interaction is to be detected by using the principle of surface plasmon resonance.

Incidentally, the detecting surface 1 is not always restricted to the flat surface 11; however, it includes a surface (and some regions or space thereon) of a variety of substrates including particles (such as plastic beads), films, and fibers.

The detecting surface 1 has double-stranded DNA 2 previously immobilized thereon. The double-stranded DNA 2 may be arrayed on the detecting surface 1 according to need. The double-stranded DNA 2 immobilized on the detecting surface 1 is a complementary double-stranded chain of deoxyribonucleic acid, which is composed of one single-stranded DNA 21, with one end thereof fixed to the detecting surface 1, and one single-stranded DNA 22, which has the base sequence complementary to the single-stranded DNA 21. (See Fig. 1.)

The double-stranded DNA 2 may be fixed to the detecting surface 1 in any manner without specific restrictions. However, the detecting surface should usually have its surface adequately treated to ensure immobilization of the double-stranded DNA 2.

Surface treatment with streptoavidin is suitable for immobilization of biotinized DNA terminals. Also, surface treatment with thiol (SH) groups is suitable for immobilization of DNA having thiol-group modified terminals through the disulfide linkage (-S-S- linkage).

The double strand DNA 2 has a specific part of the base sequence at which there is a response element 3 with which the detecting substance D combines. Incidentally, the present invention does not exclude an embodiment in which the double-stranded DNA 2 is the response element 3 itself.

Fig. 1 shows an example in which the detecting substance D is already attached to the response element 3 at the outset of assay. Fig. 1 also schematically shows the mechanism that brings about interactions between the detecting substance D and the target substance T, which has been added, injected, or infused.

In addition, Fig. 1 shows an example, which relies on the following mechanism. When the target substance T is combined with the detecting substance D attached to the response element 3 of the double-stranded DNA 2, the double-stranded DNA 2 undergoes transcriptional activation due to transcription factor. This mechanism causes the double-stranded DNA 2 to dissociate into the single-stranded DNA 21 and 22 in the downstream.

The mechanism shown in Fig. 1 is exemplified by the case in which the target substance T is a hormone or a hormone-like endocrine disrupting chemical, and the detecting substance D is a hormone receptor independent of the target substance T as a ligand.

In this case, the double-stranded DNA 2 on the detecting surface 1 has a hormone receptor capable of specific combination with retinoid (hormone), which is previously attached thereto, and the double-stranded DNA 2 undergoes transcription activation, thereby dissociating into the single-stranded DNA 21 and 22, as soon as a complex is formed from the retinoid and the hormone receptor.

Fig. 2 shows the concept of another fundamental mechanism governing the present invention. This mechanism works as follows. The target substance T reacts with the detecting substance d₁ to give another detecting substance d₂, which differs from the former in three-dimensional structure, and it combines with the response element 3 in the double-stranded DNA 2.

The sample shown in Fig. 2 is based on the mechanism working as follows. The double-stranded DNA 2 undergoes transcriptional activation due to transcription factor in response to combination between the detecting substance d₂ and the response element 3, and then it dissociates into the single-stranded DNA 21 and 22 in the downstream.

The mechanism shown in Fig. 2 is exemplified by the case in which the target substance T is a hormone or a hormone-like endocrine disrupting chemical and the detecting substance d₁ is a hormone receptor dependent on the target substance T as a ligand.

In this case, hormone response element (equivalent to double-stranded DNA) in the promoter region of estrogen-responsive gene is previously fixed to the detecting surface 1. The hormone response element is given estrogen (hormone) and estrogen receptor α (equivalent to the detecting substance d₁) so that they react with each other. This reaction changes the estrogen receptor α in three-dimensional structure, thereby causing it to combine with the hormone response element. Thus the estrogen-responsive gene undergoes transcription activation due to transcription factor, thereby dissociating into single-stranded DNA.

Now, L. L. Looger et al. found that five receptors (proteins each combined with glucose, ribose, arabinose, glutamine, and histidine) of Escherichia coli, which have been modified such that they specifically combine with molecules differing from natural ligands. The receptors indicate that (i) they have an outstanding specificity (which permits detection of similar molecular structure and optical isomers), (ii) they have approximately the same affinity as natural ligand, and (iii) they retain their functionality even after they have been made into a complex. Moreover, they also showed that it was possible to narrow down the candidates within a practical length of time. (L.L. Looger, M.A. Dwyet, J.J. Smith, and H.W.Hellinga: "Computational design of receptor and sensor proteins with novel functions", Nature, 423, 185-190 [2003])

The above-mentioned article by L. L. Looger et al. suggests the possibility that the receptor will specifically combine with a different ligand while retaining its function to react with a ligand to dissociate double-stranded DNA 2 into single-stranded DNA. This means that the receptor will be able to react with an artificially selected target substance T while retaining its inherent functions. In other words, the receptor may be made to function as the detecting substance. Thus the mechanism shown in Figs. 1 and 2 is feasible by those who are skilled in the art.

It is considered theoretically possible to modify the above-mentioned detecting substance D or d₁, which functions as a receptor, such that when it reacts with the target substance T as a ligand and combines with the response element 3 of the double-stranded DNA 2, it activates the transcription of the double-stranded DNA 2, thereby bringing about dissociation into the single-stranded DNA. Thus the mechanism shown in Figs. 1 and 2 is feasible by those who are skilled in the art.

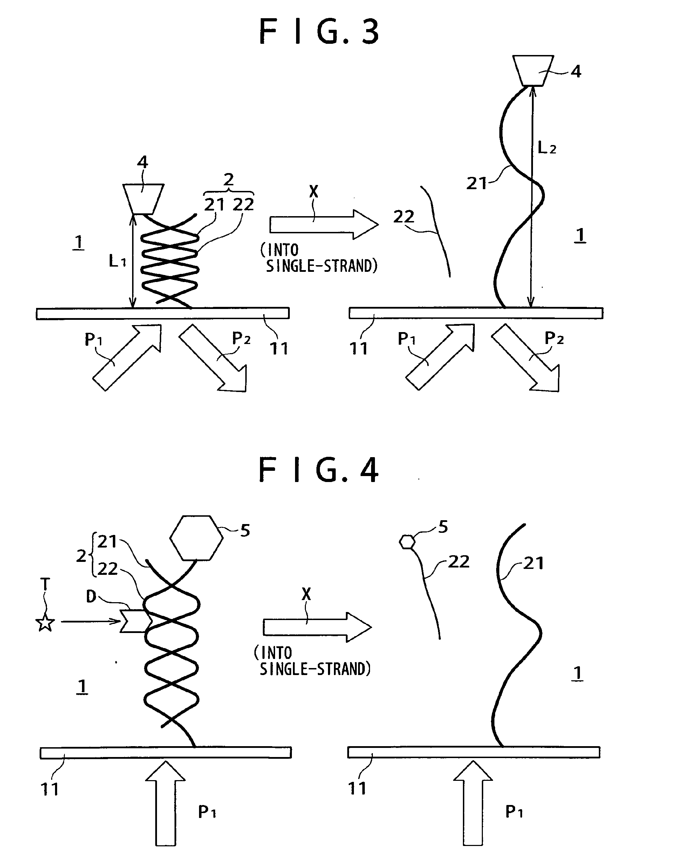

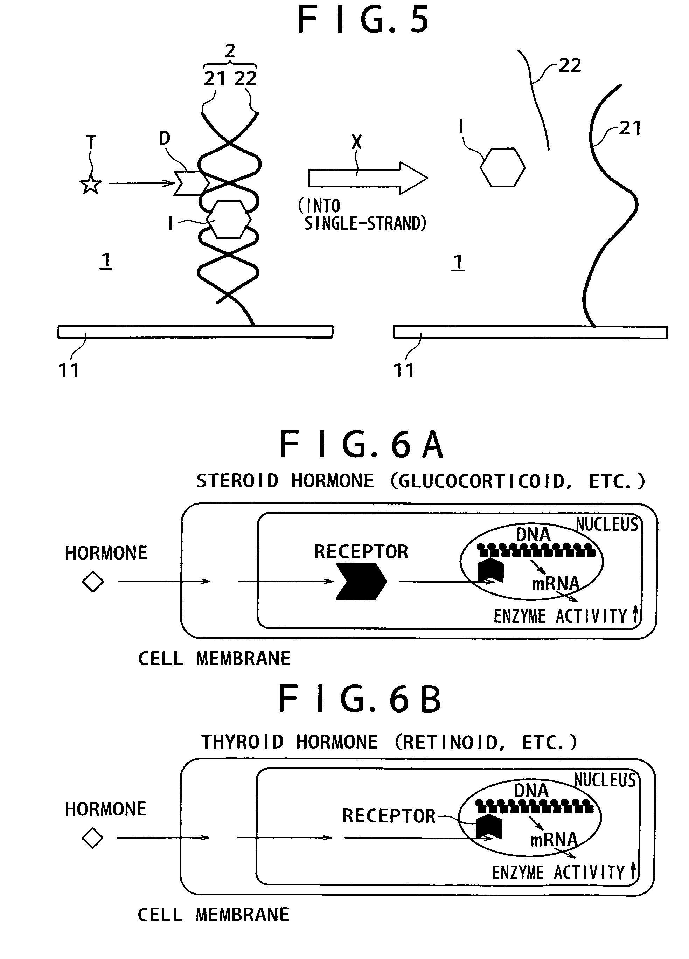

Fig. 3 is a schematic diagram illustrating the concept of the first embodiment suitable for detection of dissociation of the double-stranded DNA 2 into the single-stranded DNA. Fig. 4 is a schematic diagram illustrating the concept of the second embodiment for the same purpose as above. Fig. 5 is a schematic diagram illustrating the concept of the third embodiment for the same purpose as above.

The first embodiment shown in Fig. 3 will be described in the following.

The detecting surface 1 formed on the substrate 11 has the double-stranded DNA 2 in such a way that the single-stranded DNA 21 thereof is fixed thereto. The upper end of the immobilized single-stranded DNA 21 is labeled with a dielectric substance 4 (such as dielectric crystal).

The double-stranded DNA 2 has the response element 3, with which the detecting substance D (or d₂), such as hormone receptor, reacts. In response to this reaction, the double-stranded DNA 2 dissociates into the single-stranded DNA 21 and 22 through transcriptional activation.

The process of this reaction changes the distance between the substrate 11 and the dielectric substance 4. To be concrete, the distance L₂ (after dissociation) is larger than the distance L₁ (before dissociation), or L₁ < L₂. The change in distance changes the dielectric constant and hence changes the peak of intensity of reflected rays. This change is detected, and it is possible to judge whether or not an interaction took place between the detecting substance and the response element. Incidentally, the incident ray and reflected ray used for detection are indicated by P₁ and P₂, respectively, in Fig. 3.

The second embodiment shown in Fig. 4 will be described in the following.

The detecting surface 1 formed on the substrate 11 has the double-stranded DNA 2 in such a way that the single-stranded DNA 22 thereof is fixed thereto. The upper end of the immobilized single-stranded DNA 22 is labeled with a fluorescent substance 5 (such as fluorescent dye called Cy-3 or Cy-5, available from Amersham Pharmacia Inc.).

The double-stranded DNA 2 has the response element 3, with which the detecting substance D (or d₂), such as hormone receptor, reacts. In response to this reaction, the double-stranded DNA 2 dissociates into the single-stranded DNA 21 and 22 through transcriptional activation.

As the result, the labeled single-stranded DNA 22 releases itself from the detecting surface 1, and the peak of intensity of transmitted light disappears. This disappearance is detected, and, it is possible to judge whether or not an interaction took place between the target substance T and the detecting substance D (or D₁). Incidentally, the incident light (such as laser beam to detect interaction) is indicated by P₁ in Fig. 4.

The third embodiment shown in Fig. 5 will be described in the following.

The detecting surface 1 formed on the substrate 11 has the double-stranded DNA 2. The double-stranded DNA 2 has a fluorescent intercalator I inserted thereinto. The intercalator I may be any of POPO-1, TOTO-3, and SYBR (registered trademark) Green I.

The double-stranded DNA 2 has the response element 3, with which the detecting substance D (or d₂), such as hormone receptor, reacts. In response to this reaction, the double-stranded DNA 2 dissociates into the single-stranded DNA 21 and 22 through transcriptional activation.

As the result, the fluorescent intercalator I releases itself from the detecting surface 1, and hence its color development changes. This change is optically detected, and it is possible to judge whether or not an interaction took place between the target substance T and the detecting substance D (or d₁) .

The fourth embodiment will be described in the following with reference to Figs. 6A to 11.

This embodiment is based on the principle that when a living organism receives a stress by a stressor from the external environment, the adrenal cortex releases a steroid hormone called glucocorticoid (lipid-soluble small hormone) into blood. It combines with a water-soluble transport protein and moves in blood, so that it directly acts on the receptor in the cell.

Fig. 6A is a schematic diagram illustrating how a hormone (such as glucocorticoid) works. It should be noted that a hormone (H) combines with a receptor (R) to form a complex, which migrates into the nucleus and acts on the hormone response element of DNA [which is GRE (Glucocorticoid Reaction Element) in this case). The complex promotes an expression of a DNA-coded protein in the downstream. It is known that thyroid hormone, retinoid, vitamin D, or the like migrates into the nucleus and the acts on the receptor. (See Fig. 6B.)

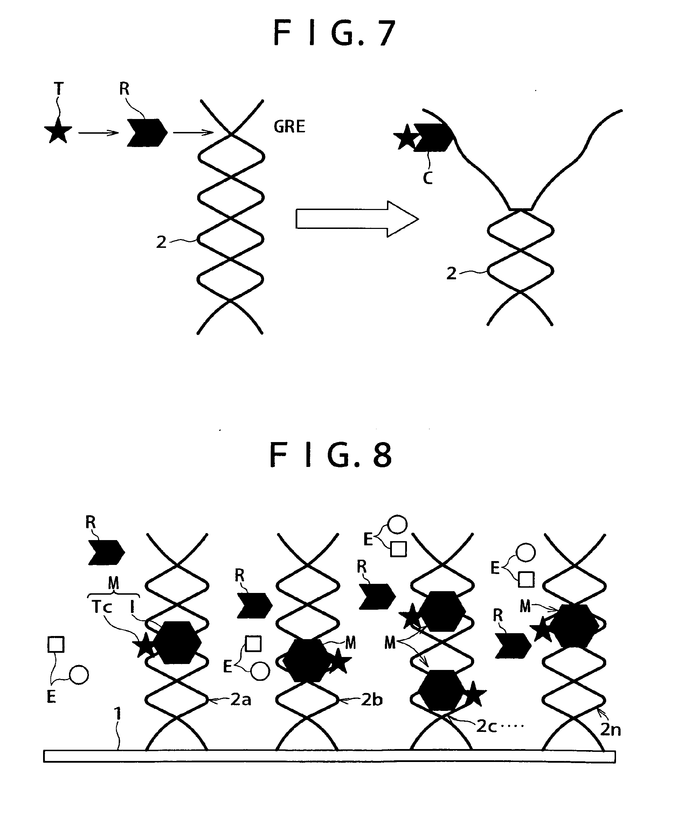

As known well, expression of protein is triggered by dissociation of double strands of DNA into single strands and subsequent transcription induced by the transcription factor. Fig. 7 is provided to help understand the present invention. It schematically shows how the double-stranded DNA 2 dissociates into single strands when the hormone-receptor complex C acts on the above-mentioned GRE of the double-stranded DNA 2.

The intercalator I is sometimes used in the conventional technology to detect hybridization. The fourth embodiment is characterized in using the intercalator I in combination with the reaction of dissociation of the double-stranded DNA into single strands which takes place at the time of transcription in response to a hormone.

In what follows, the detecting surface of the fourth embodiment will be described in more detail with reference to Fig. 8. The fourth embodiment is an assay system, which would be used for detection of cortisol (one species of glucocorticoid). However, the scope of the present invention is not restricted to such an assay system. This embodiment is based on the assumption that detection will be accomplished by SPR (Surface Plasmon Resonance). However, the scope of the present invention is not restricted to such a detecting method.

According to the fourth embodiment, the detecting surface 1 is coated with a thin metal film suitable for assay by SPR. A large number of double-stranded DNA molecules 2a, 2b, 2c, ... 2n (each having GRE) are fixed to the thin metal film. A conjugate M of cortisol Tc (as a target substance to be detected) and an intercalator I, which has been prepared previously, is intercalated into the double-stranded DNA 2. A medium is introduced onto the detecting surface 1 and subsequently it is incorporated with a cortisol receptor R and a transcription factor E required. (See Fig. 8.)

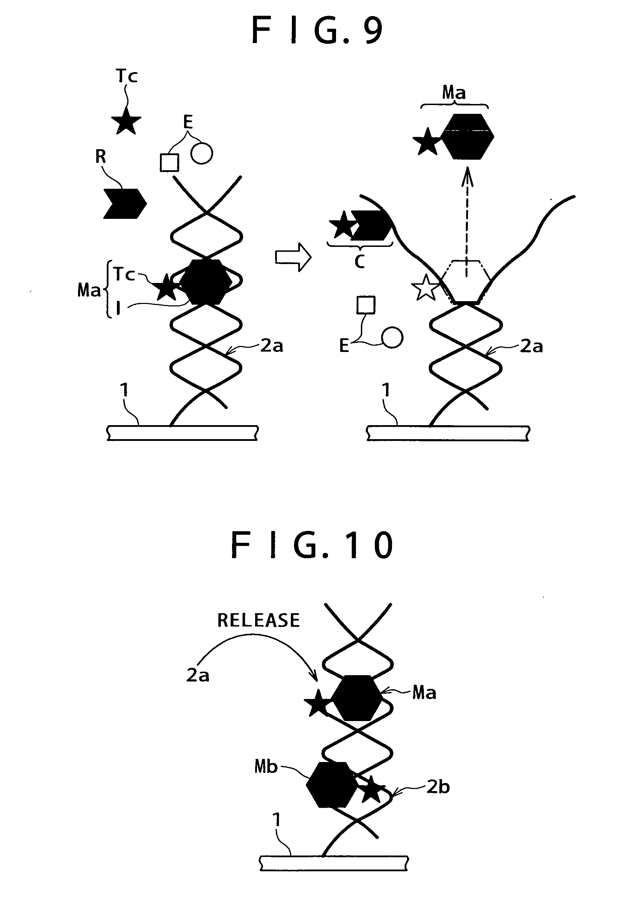

Upon introduction into the region on the detecting surface 1, cortisol Tc specifically combines with the receptor R (in free state) to form the complex C. This complex C (in the form of dimer) acts on the GRE in the double-stranded DNA (such as the one denoted by 2a). This action triggers dissociation of the double-stranded DNA 2a into single strands in concert with the transcription factor E. (See Fig. 9.)

In the course of dissociation into single strands, the conjugate Ma, which has been intercalated into the double-stranded DNA 2a, releases itself into the medium (as indicated by an arrow of dotted line in fig. 9). The released conjugate Ma intercalates itself into the complementary base pair of the double-stranded DNA 2b, which remains in the state of double strand and remains fixed to the detecting surface 1 to which the double-stranded DNA 2a has been fixed. See Fig. 10, which shows what occurs after the conjugate Ma released from a first double-stranded DNA 2a has intercalated itself into the complementary base pair in a second double-stranded DNA 2b. The reference numeral Mb in Fig. 10 indicates the conjugate previously intercalated in the double-stranded DNA 2b.

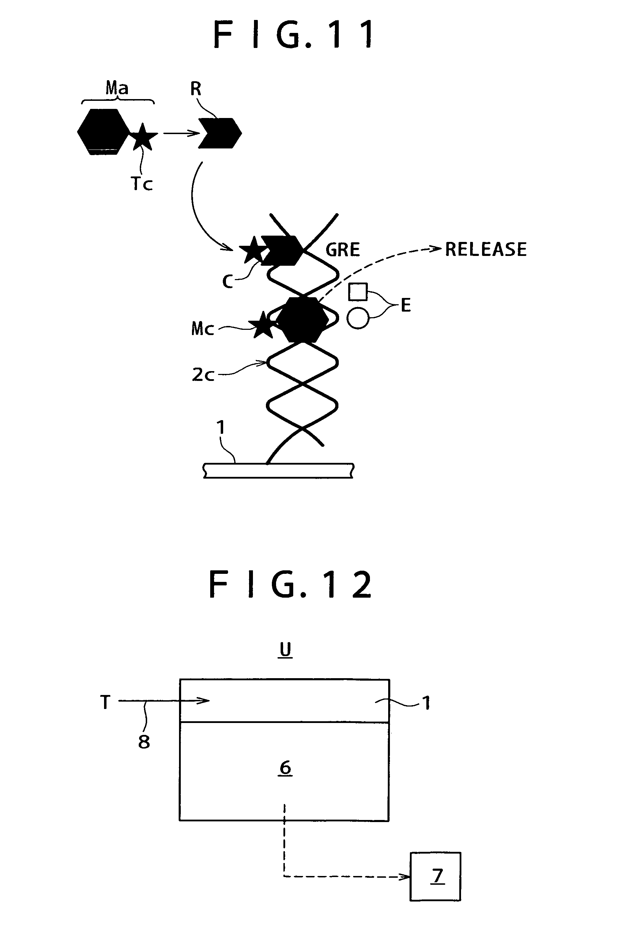

Another reaction may take place as follows. The conjugate Ma, which has been released from the double-stranded DNA 2a at the time of dissociation into single strands, combines with the receptor R, in which the cortisol Tc constituting the conjugate Ma remains in a free state, thereby forming the hormone-receptor complex C. This complex C acts on the GRE of another double-stranded DNA 2c. This action triggers dissociation of the double-stranded DNA 2c into single strands in concert with the transcription factor E. (See Fig. 11.)

In the course of dissociation into single strands, the conjugate Mc, which has been intercalated into the complimentary base pair of the double-stranded DNA 2c, releases itself into the medium. The released conjugate Mc behaves in the same way as the above-mentioned conjugate Ma. Tt intercalates itself into the complementary base pair of the other double-stranded DNA, or it combines with receptor R to form the hormone-receptor complex C, which acts on the GRE of the other double-stranded DNA, thereby starting dissociation into single strands in concert with the transcription factor E. (See Fig. 11.)

As explained above, the molecules (2a to 2n) of double-stranded DNA are made to sequentially dissociate into single strands by the foregoing process which includes regularly fixing the molecules (2a to 2n) of double-stranded DNA onto the detecting surface 1, previously intercalating the conjugate M of the target substance T (such as hormone) to be detected and the intercalator I into each of the molecules (2a to 2n) of double-stranded DNA, and introducing the target substance T to be detected into the double-stranded DNA.

The sequential dissociation into single strands exponentially amplifies the initial signal of the sample, thereby allowing sensing with high sensitivity. Incidentally, the change of double-stranded DNA into single strands may be measured in terms of change in dielectric constant by SPR. However, any method other than SPR may be employed. For example, it is possible to use a fluorescent intercalator as the intercalator I and measure fluorescence that changes when the fluorescent intercalator passes from the intercalated state to the free state.

As mentioned above, it is possible to detect or measure interaction between substances by the process including a step of fixing previously prepared double-stranded DNA 2 to the detecting surface, a step of causing the double-stranded DNA 2 to dissociate into the single-stranded DNA 21 and 22 in response to interaction between substances, and a step of detecting the dissociation.

The above-mentioned detecting surface 1 may be used to produce a sensor chip or a microarray based on the existing known technology. It is also possible to produce a sensing device including the detecting surface 1 and a detecting means capable of detecting dissociation of the double-stranded DNA 2 into the single-stranded DNA 21 and 22 that takes place on the detecting surface 1. This sensing device includes those devices categorized as biosensor. In this case, the detecting surface 1 may be interpreted as a means to provide a "bioelement".

Fig. 12 is a simplified diagram showing the basic structure of the sensing device indicated by a reference character U. This sensing device is equipped with at least a detecting surface 1 (or a sensor chip (not shown) having the detecting surface 1) and a detection principle unit 6 capable of detecting the change in the peak of intensity of reflected light which occurs when the dielectric constant changes on the detecting surface 1 (as in the first embodiment shown in Fig. 3), detecting the disappearance of the peak of intensity of transmitted light (as in the second embodiment shown in Fig. 4), or detecting the change in color development (as in the third embodiment shown in Fig. 5). The sensing device also includes an analyzing unit 7 to analyze data from the detecting unit 6 and a micro flow system 8 to supply the sample at a constant flow rate.

The detection principle unit 6 may include any of laser beam irradiating apparatus, confocal lens and confocal scanning apparatus, a CCD camera, means based on the plasmon resonance principle, means to detect the change in frequencies of a quartz oscillator, and transducer, which should be properly selected according to the principle of detection.

The sensing device U works in the following manner. The detecting surface 1 is given a sample containing a target substance T by injection, dropping, or the like, so that interaction takes place between the target substance T and the detecting substance D. Then, this interaction is detected by the detecting unit 6 and examined by the data analyzing unit 7.

The present invention will be applied to the assay technique of detecting interaction between substances and also to the sensing device (including sensor chip, microarray, and biosensor) that utilizes the reaction of living organisms, such as hormone response reaction.

The present invention will be applied to the screening of new drugs and endocrine disrupting chemicals. It will also be applied to kinetics analysis, affinity analysis, functional analysis of multimolecular complex, analysis of structure-function correlation, and analysis of specificity of bonding.

The present invention will also be applied to analysis of bioinformation, such as genomics, transcriptome analysis for genome to transcription, proteome analysis for expression proteins which are translated and produced in living organisms and cells, metabolome analysis for metabolism, and signalome analysis for signals in living organisms. It will be applied to the sensor technique for measuring, examining, or diagnosing emotion and stress.

While preferred embodiments of the invention have been described using specific terms, such description is for illustrative purposes only, and it is to be understood that changes and variations may be made without departing from the spirit of scope of the following claims.

## Claims

1. A detecting surface (1) for detection of interaction between substances, said detecting surface comprising double-stranded DNA (2) which forms the complimentary linkage with single-stranded DNA (21, 22) having one end thereof fixed to said detecting surface (1), such that said double-stranded DNA dissociates into single-stranded DNA (21, 22) in response to said interaction.

2. The detecting surface (1) as defined in Claim 1, wherein the interaction is one which takes place between a target substance introduced onto the detecting surface (1) and a detecting substance (D) that acts directly or indirectly on a specific site of sequence of the double-stranded DNA.

3. The detecting surface (1) as defined in Claim 2, wherein the target substance is a hormone, the detecting substance is a hormone receptor, and the specific site of sequence is a hormone response element.

4. The detecting surface (1) as defined in Claim 2, wherein the detecting substance is a transcription activation factor and the dissociation of double-stranded DNA into single-stranded DNA is the change of state resulting from transcription.

5. The detecting surface (1) as defined in Claim 2, wherein the detecting substance is a substance which, upon interaction with the target substance present on the detecting surface, changes in three-dimension structure and acts on the specific site of sequence.

6. The detecting surface (1) as defined in Claim 4, wherein the target substance is a hormone, the detecting substance is a ligand-dependent hormone receptor, and the specific site of sequence is a hormone response element.

7. The detecting surface (1) as defined in one of the Claims 1 to 6, wherein the double-stranded DNA has a fluorescent intercalator inserted thereinto.

8. The detecting surface (1) as defined in one of the Claims 1 to 6, wherein the DNA strand, which is not fixed to the detecting surface (1), has its end labeled with a fluorescent substance.

9. The detecting surface (1) as defined in one of the Claims 1 to 6, wherein the DNA strand, which is fixed to the detecting surface, has its end labeled with a dielectric substance.

10. The detecting surface (1) as defined in Claim 2, wherein the double-stranded DNA (2) has a complex composed of the target substance and the intercalator, which binds to the site of the complementary base pair.

11. The detecting surface (1) as defined in Claim 10, wherein the target substance is a hormone.

12. A sensor chip comprising the detecting surface defined in one of the Claims 1 to 11.

13. A sensing device comprising the detecting surface defined in one of the Claims 1 to 11 and a means of detecting the dissociation of the double-stranded DNA into the single-stranded DNA, which occurs on the detecting surface.

14. A method of detecting an interaction between substances comprising a step of fixing double-stranded DNA to a detecting surface, a step of causing the double-stranded DNA to dissociate into the single-stranded DNA in response to interaction between substances, and a step of detecting said dissociation.

15. The method as defined in Claim 14, further comprising a step of binding a conjugate composed of a target substance to be detected and an intercalator to the site of the complementary base pair of the double-stranded DNA.

16. The method as defined in Claim 15, further comprising a step in which the conjugate releases itself into the medium on the detecting surface when the double-stranded DNA dissociates into the single-stranded DNA, and a step in which the target substance constituting the released conjugate promotes the dissociation through combination with a receptor.

17. The method as defined in Claim 15, wherein the target substance is a hormone.

18. The process as defined in one of the Claims 14 to 17, wherein the detection is accomplished by the procedure based on the principle of Surface Plasmon Resonance.

19. The method as defined in one of the Claims 14 to 18, wherein the detection is accomplished by the procedure which is designed to detect, based on the principle of Surface Plasmon Resonance, the change in dielectric constant that occurs when the distance between the detecting surface and the dielectric substance labeling the end of the fixed DNA strand of the double-stranded DNA changes as the double-stranded DNA dissociates into the single-stranded DNA.

20. The method as defined in one of the Claims 14 to 19, wherein the detection is accomplished optically by measuring either of the following two items (1) or (2).
(1) Change in intensity of fluorescence of the fluorescent substance labeling the double-stranded DNA.
(2) Change in color development of the fluorescent intercalator inserted into the double-stranded DNA.
